(19) Europäisches Patentamt
European Patent Office
Office européen des brevets

(11) Numéro de publication: **0 563 227 B1**

(12) **FASCICULE DE BREVET EUROPEEN**

(45) Date de publication de fascicule du brevet: **11.01.95**

(51) Int. Cl.[6]: **A61K 7/00**, A61K 7/06, A61K 47/36, A61K 47/32

(21) Numéro de dépôt: **92902320.8**

(22) Date de dépôt: **19.12.91**

(86) Numéro de dépôt internationale : **PCT/FR91/01037**

(87) Numéro de publication internationale : **WO 92/10989 (09.07.92 92/17)**

Le dossier contient des informations techniques présentées postérieurement au dépôt de la demande et ne figurant pas dans le présent fascicule.

(54) **COMPOSITION COSMETIQUE OU PHARMACEUTIQUE SE PRESENTANT SOUS LA FORME D'UN GEL RIGIDE, NOTAMMENT EN VUE DU MAINTIEN D'INCLUSIONS EN SON SEIN.**

(30) Priorité: **21.12.90 FR 9016099**

(43) Date de publication de la demande: **06.10.93 Bulletin 93/40**

(45) Mention de la délivrance du brevet: **11.01.95 Bulletin 95/02**

(84) Etats contractants désignés: **BE CH DE FR GB IT LI**

(56) Documents cités:
**EP-A- 0 177 223**
**EP-A- 0 336 900**
**EP-A- 0 346 097**

**Patent Abstracts of Japan, Vol. 9, no. 171 (C-291) 1984, 16.07.85, & JP-A-6045511 (Kobayashi Koosee K.K.) 12.03.85**

(73) Titulaire: **L'OREAL**
**14, Rue Royale**
**F-75008 Paris (FR)**

(72) Inventeur: **NADAUD, Jean-François**
**76, boulevard Arago**
**F-75013 Paris (FR)**

(74) Mandataire: **Michardière, Bernard et al**
**Cabinet Peuscet**
**68, rue d'Hauteville**
**F-75010 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 563 227 B1

Rank Xerox (UK) Business Services
(3.10/3.09/3.3.3)

## Description

La présente invention concerne une composition cosmétique ou pharmaceutique, qui se présente sous la forme d'un gel qui reste rigide au cours du stockage, même lorsqu'il est contenu dans des pots ou similaires de grand volume mais qui est susceptible de redevenir fluide lors de l'application en donnant un produit de qualité cosmétique, c'est-à-dire doux et non collant.

Il est apparu récemment sur le marché, des compositions cosmétiques renfermant deux phases visqueuses (A) et (B), la phase (A), dite interne, étant disposée sous la forme d'au moins une inclusion au sein de la phase (B), dite externe. La phase (A) peut se présenter sous la forme d'une formulation aqueuse épaissie ou gélifiée, d'une émulsion huile-dans-eau ou eau-dans-huile, d'une dispersion aqueuse de vésicules constituées de feuillets lipidiques ioniques ou non-ioniques encapsulant une phase aqueuse, ou encore de cristaux liquides. Par exemple, on peut injecter, dans des gels transparents, des produits colorés réalisant des dessins très variés à l'intérieur du gel. Dans ce cas, il est important que la phase externe soit parfaitement rigide et stable, afin d'éviter toute déformation du dessin ou du graphisme que forme(nt) la (ou les) inclusion(s), lorsque le produit est transporté ou manipulé.

Par ailleurs, d'une manière générale, on peut, dans certaines applications, être amené à formuler des gels transparents, incolores, stables et rigides.

Par US-A 4 143 007, on connaît une composition constituée par un mélange d'une gomme de polygalactomannane et d'un copolymère à base d'un anhydride d'acide dicarboxylique à insaturation alpha, bêta-oléfinique et d'un comonomère choisi parmi les hydrocarbures à insaturation alpha-oléfinique et les alkyl-vinyl éthers. Cette composition est décrite comme apportant une viscosité exceptionnellement élevée à une solution aqueuse, en raison d'un effet de viscosité synergique provenant de l'interaction du polygalactomannane et des composants du copolymère. Par US-A 3 658 734, on connaît également une composition comprenant un mélange de gomme de guar et d'un polyacrylamide, décrite comme apportant, lorsqu'elle est ajoutée à un milieu aqueux, une augmentation synergique de la viscosité du milieu. Toutefois, en pratique, les mélanges de polymères des deux brevets américains précités, connent des gels opaques et troubles et qui restent fluides. Le document EP-A 0 346 097 décrit un système liquide contenant un mélange épaississant formé d'un mélange d'un polymère de type gomme et d'un polymère de type acrylique. Ce mélange a l'avantage de donner un gel ayant une viscosité élevée ; mais il n'est pas précisé dans ce document que certains de ces mélanges permettent d'obtenir des gels restant rigides même lorsqu'ils sont contenus dans des pots ou similaires de grand volume, par exemple supérieur à 50 ml.

Il est connu par JP-A-60-45511 d'utiliser comme gélifiant un mélange d'un polymère soluble dans l'eau, qui peut être la gomme de guar ou un polyacrylate de sodium avec un polymère cationique.

EP-A-0 177 223 décrit une composition pharmaceutique contenant des vésicules de lipides encapsulant un actif, les vésicules étant sous forme de dispersion dans une phase aqueuse saturée en actif et contenant l'actif sous forme solide. La phase aqueuse peut contenir, entre autres, un hydrocolloïde qui n'est pas un polygalactomannane.

Les agents épaississants et/ou gélifiants, qui sont les plus adaptés à la préparation de gels transparents, incolores, sont les polymères carboxyvinyliques, très connus et largement utilisés notamment dans le domaine cosmétique. A titre d'exemples, on peut mentionner les acides polyacryliques réticulés, commercialisés sous les dénominations "CARBOPOL 934, 940 et 941" par la Société GOODRICH. Si on introduit un polymère carboxylique seul, pour obtenir un seuil d'écoulement et une consistance permettant de préparer un produit rigide qui peut être stocké, on est obligé d'ajouter une quantité de polymère carboxylique telle que le produit obtenu n'est plus cosmétique lors de l'application.

La Société déposante a recherché une solution à ce problème, et elle a découvert, de façon surprenante, que l'association d'au moins un polygalactomannane, à un très faible taux, à un gel obtenu avec un polymère à base de monomère carboxyvinylique comme agent gélifiant, permet d'augmenter le seuil d'écoulement et la consistance, c'est-à-dire la rigidité, suffisamment pour que l'on puisse obtenir des gels rigides qui conservent cette caractéristique même lorsqu'ils sont contenus dans des pots ou similaires, de grand volume, notamment supérieur à 50 ml et qui, lors de l'application, donnent par cisaillement un produit doux et non collant ; en d'autres termes, lors de l'application, le produit n'est pas trop visqueux, ce que ne permettait pas de prévoir les résultats constatés avec les compositions de US-A 4 143 007, US-A 3 658 734 et EP-A 0 346 097. Il est alors possible de préparer des gels rigides, quasi-solides, comportant éventuellement en leur sein des inclusions stables de phases liquides ou visqueuses, de tels gels étant contenus dans des pots ou similaires, de volume important, allant par exemple jusqu'à environ 250 ml. Ces gels peuvent ensuite être appliqués sur la peau sans donner par cisaillement un gel trop visqueux, qui serait collant et ne serait donc pas utilisable en cosmétique. Selon le procédé de l'invention, on a donc une

augmentation du seuil d'écoulement sans augmentation gênante de la viscosité après cisaillement. Il faut noter que ce résultat ne peut pas être obtenu avec des polysaccharides autres que les polygalactomannanes, comme les gommes de xanthane. Par ailleurs, la Société déposante a découvert, de façon tout aussi surprenante, que cette association permet de corriger ou de limiter l'incompatibilité avec certains sels des gels à base de tels polymères, incompatibilité qui se manifeste même si ces polymères sont neutralisés.

Comme polygalactomannane, on peut utiliser la gomme de caroube, la gomme de guar et la gomme de tara. On utilise de préférence la gomme de caroube.

La gomme de caroube est un produit bien connu et utilisé dans les produits alimentaires et cosmétiques. Les solutions de gomme de caroube, jusqu'à 0,5 % en poids, se présentent sous une forme liquide. A 1 % en poids, le liquide est légèrement visqueux, voire gélatineux. La gomme de caroube est connue comme pouvant influencer les propriétés gélifiantes des gels de carraghénane et d'agar, dans le but de rendre ces derniers plus élastiques et d'accroître leur force de rupture. Elle a, par ailleurs, été associée à d'autre gommes, par exemple, à la gomme de xanthane, en particulier pour former un agent liant utilisable en pharmacie (JP-A 79/070 420), pour former un milieu gélifié pour le traitement des brûlures (FR-B 2 085 737), ou pour former des compositions gélifiables et gélifiées contenant de l'agar associé à une combinaison xanthane-caroube (US-A 3 700 451 et US-A 3 944 427), et à la gomme de guar, en particulier, pour former des compositions cosmétiques de nettoyage (JP-B 85/112 712, JP-B 85/197 798 et JP-B 85/197 799). Les gommes de tara, de guar et de caroube sont connues dans leurs associations avec un alginate de calcium ou d'aluminium, en particulier pour former des lotions aqueuses cosmétiques (US-A 3 764 707).

Par ailleurs, on peut mentionner que, dans la demande de brevet japonais JP-A 87/039 524, on envisage l'introduction, dans un onguent anti-inflammatoire, d'un agent gélifiant et/ou épaississant choisi dans un groupe comprenant, entre autres, la gomme de caroube et les polymères à base de monomères carboxyvinyliques. Cependant, dans les exemples de cette demande de brevet, on ne retrouve jamais associés un polymère carboxyvinylique et la gomme de caroube.

Enfin, on connaît, par le brevet européen EP-B 67 025, un produit liquide de nettoyage dans lequel sont associés un agent tensio-actif, de la gomme de guar et un polymère à base de monomère carboxyvinylique. Bien que l'association d'un galactomannane avec un tel polymère soit décrite dans ce brevet, la présence d'un agent tensio-actif modifie la nature du problème à résoudre. En effet, l'instabilité des compositions de nettoyage à base de gomme de guar, décrite dans ce brevet, instabilité qui est évitée grâce à l'addition dudit polymère, est due à la présence de l'agent tensio-actif. Par ailleurs, il s'agit de compositions liquides, et il n'est donc jamais fait mention de propriétés rhéologiques particulières de l'association.

La demanderesse a maintenant constaté que, de façon inattendue, en combinant un polygalactomannane aux polymères carboxyvinyliques, des modifications inattendues des propriétés rhéologiques de ces derniers apparaissent, lesquelles permettent de résoudre les problèmes décrits précédemment, problèmes qui résultaient de la mise en oeuvre desdits polymères carboxyvinyliques utilisés seuls. Les gels obtenus sont transparents et ils présentent des caractéristiques cosmétiques tout à fait satisfaisantes lors de l'application. Ils ont un comportement rhéologique étonnant : après 48 heures environ de repos, ils se comportent comme un solide, au sens rhéologique du terme, jusqu'à la contrainte seuil, et la transition solide/liquide est, pour ces gels, beaucoup plus élevée que pour la moyenne des gels. Par conséquent, ils peuvent stabiliser physiquement toutes les phases en suspension, même pour des volumes importants. Par ailleurs, la présence de sels dans le milieu ne perturbe pas l'état rhéologique du gel. Il en résulte qu'une telle association polymère carboxyvinylique/galactomannane permet d'élargir le domaine d'application des polymères du type précité.

La présente invention a donc d'abord pour objet une composition cosmétique ou pharmaceutique, qui comprend au moins une phase gélifiée (ou rigidifiée), l'une au moins desdites phases comportant au moins un polymère carboxyvinylique en tant qu'agent gélifiant, caractérisée par le fait qu'audit polymère carboxyvinylique est associé au moins un polygalactomannane.

Le polygalactomannane est choisi notamment dans le groupe formé par la gomme de caroube, la gomme de guar et la gomme de tara. Il est, de préférence la gomme de caroube.

Le polymère à base de monomère carboxyvinylique est, de façon préférée, un acide polyacrylique réticulé, de masse moléculaire comprise entre 800 000 et 5 000 000.

Conformément à la présente invention, le(s) polymère(s) carboxyvinylique(s) est (ou sont) présent(s) notamment à raison de 0,01 à 2 % en poids, de préférence de 0,1 à 1,0 % en poids, de la phase gélifiée qui le(s) contient. Quant au(x) polygalactomannane(s), il(s) représente(nt) notamment de 0,01 à 3 % en poids, de préférence de 0,01 à 1,5 % en poids, de la phase gélifiée qui le(s) contient.

On ajoute plus particulièrement une quantité de polygalactomannane telle que l'indice de consistance du polymère carboxyvinylique soit augmenté d'au moins 10 % par rapport à la valeur obtenue avec le polymère carboxyvinylique seul.

La composition selon l'invention peut renfermer des sels normalement incompatibles avec les polymères carboxyvinyliques. Parmi ces sels, on peut citer les sels organiques tels que le salicylate de triéthanolamine, le benzoate de sodium et le salicylate de sodium.

Conformément à un mode de réalisation, la composition selon l'invention comporte une phase gélifiée grâce au(x) polymère(s) carboxyvinylique(s) et au(x) polygalactomannane(s) dans laquelle se trouve au moins un ingrédient cosmétiquement ou pharmaceutiquement actif et/ou au moins un additif cosmétiquement ou pharmaceutiquement acceptable.

Conformément à un autre mode de réalisation, la composition selon l'invention consiste en au moins deux phases, l'une étant une phase continue dite "externe", gélifiée grâce au(x) polymère(s) carboxyvinylique(s), chaque autre phase étant une phase dite "interne", sous forme d'un liquide plus ou moins visqueux, se présentant sous la forme d'au moins une inclusion au sein de ladite phase externe ; une phase interne peut être constituée par une formulation aqueuse liquide, épaissie ou gélifiée, par une émulsion huile-dans-eau ou eau-dans-huile, par une dispersion aqueuse de vésicules constituées de feuillets lipidiques ioniques ou non-ioniques encapsulant une phase aqueuse, ou par des cristaux liquides ; ladite (ou lesdites) phase(s) interne(s) peut (peuvent) renfermer au moins un ingrédient cosmétiquement ou pharmaceutiquement actif et/ou au moins un additif cosmétiquement ou pharmaceutiquement acceptable.

Dans ce cas, la (ou les) phase(s) interne(s) représente(nt) avantageusement de 0,1 à 25 % en poids de ladite composition.

Les vésicules mentionnées ci-dessus sont bien connues et préparées à partir d'une phase lipidique comprenant au moins un lipide amphiphile ionique et/ou non-ionique associé éventuellement à au moins un additif stabilisant, et elles contiennent une phase encapsulée, laquelle peut renfermer des produits cosmétiquement ou pharmaceutiquement actifs, tels que des agents hydratants ou des agents apaisants.

Parmi les lipides non-ioniques utilisables, on peut citer :

(1) les éthers de polyglycérol, linéaires ou ramifiés, de formule :

$$R^0O-\left[C_3H_5(OH)O\right]_{\bar{n}}-H$$

dans laquelle :

- $-C_3H_5(OH)O$ est représenté par les stuctures suivantes prises en mélange ou séparément :
  $-CH_2CHOHCH_2O-$ ,

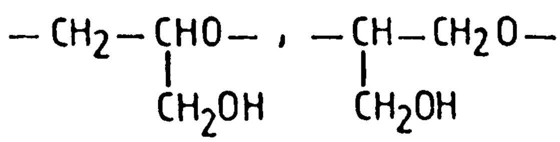

- $\bar{n}$ est une valeur statistique moyenne comprise entre 2 et 6 ;
- $R^0$ représente :
  (a) une chaîne aliphatique, linéaire ou ramifiée, saturée ou insaturée, contenant de 12 à 18 atomes de carbone ;
  (b) un reste $R^1CO$, où $R^1$ est un radical aliphatique, linéaire ou ramifié, en $C_{11}$-$C_{17}$ ;
  (c) un reste

$$R^2-\left[OC_2H_3(R^3)\right]-$$

où :

- $R^2$ peut prendre la signification (a) ou (b) donnée pour $R^0$ ;

- $OC_2H_3(R^3)$- est représenté par les stuctures suivantes, prises en mélange ou séparément :

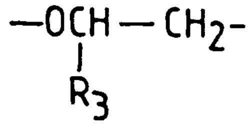 et 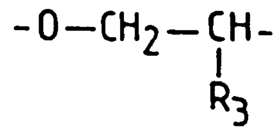

où $R^3$ prend la signification (a) donnée pour $R^0$ ;

(2) les alcools gras polyoxyéthylénés ;
(3) les esters de polyols éventuellement polyoxyéthylénés ;
(4) les cérébrosides ; et
(5) le stéarate de polyglycérol oxyéthyléné ;
(6) les stérols polyoxyéthylénés.

Parmi les lipides amphiphiles ioniques utilisables, on peut citer :

- les phosphoaminolipides ;
- les glycolipides ;
- les phospholipides naturels, tels que la lécithine d'oeuf ou de soja, la sphingomyéline, la phosphatidylsérine, dipalmitoylphosphatidylcholine et les lécithines hydrogénées.

L'additif stabilisant est destiné, de façon connue, à modifier la perméabilité et/ou la charge superficielle des vésicules. Il est, de préférence, choisi dans le groupe formé par les stérols et les stabilisants anioniques. Le stérol est avantageusement le cholestérol. Le stabilisant anionique est avantageusement choisi, d'une part, parmi les sels monosodiques ou disodiques (d'acyl en $C_{14}$-$C_{22}$) glutamates, tel que le sel monosodique de l'acide N-stéaroylglutamique, les sels disodiques avec des radicaux acyle du coprah et du suif ou encore les radicaux cocoyle et stéaroyle, et, d'autre part, parmi les esters phosphoriques d'alcools gras en $C_{12}$-$C_{22}$. De façon connue, on peut ajouter au(x) lipide(s) amphiphile(s) à la fois un stérol et un stabilisant anionique.

Dans le cas où l'une des phases internes est épaissie, le (ou les) agent(s) épaississant(s) utilisé(s) dans ce but est (ou sont) choisi(s) dans le groupe formé par les acides polyacryliques réticulés, par exemple ceux commercialisés sous la dénomination de "CARBOPOL", les dérivés de cellulose, par exemple, l'hydroxypropylcellulose, la carboxyméthylcellulose, l'hydroxyéthylcellulose, l'hydroxypropylméthylcellulose, les mélanges d'alcool cétylstéarylique et d'alcool cétylstéarylique oxyéthyléné (par exemple, à 33 moles d'oxyde d'éthylène), les copolymères acrylamide/acrylate de sodium et les copolymères vinypyrrolidone/acétate de vinyle. Ce (ou ces) agent(s) épaississant(s) est (ou sont) présent(s) généralement à raison de 1 à 10 % en poids par rapport au poids total de la phase qui est épaissie.

Les ingrédients actifs, dont il est question ci-dessus, sont choisis notamment parmi les agents amincissants, les agents hydratants, les agents de fixation dans le domaine capillaire, les agents de protection solaire et tout agent de soin de la peau et des cheveux (vitamines, oligo-éléments, extraits végétaux, etc...).

Quant aux additifs dont il est question ci-dessus, ils sont choisis notamment dans le groupe formé par les agents émollients, les parfums, les agents modificateurs de pH, les agents conservateurs et les agents séquestrants.

Un procédé de fabrication de la composition telle que définie ci-dessus est caractérisé par le fait qu'on prépare une phase gélifiée ou rigidifiée par incorporation d'une solution aqueuse d'au moins un polygalactomannane dans un gel aqueux à base d'au moins un polymère carboxyvinylique, avant ou après neutralisation de ce dernier ou de ces derniers, par exemple, par une base minérale ou organique, qu'on incorpore éventuellement, avant ou après ladite neutralisation, au moins un ingrédient cosmétiquement ou pharmaceutiquement actif et/ou au moins un additif cosmétiquement ou pharmaceutiquement acceptable, qu'on dilue à l'eau, le cas échéant, pour obtenir au bout de 1 à 5 jours, un gel transparent et rigide, dans lequel on injecte, éventuellement, au moins une phase visqueuse pour former au moins une inclusion au sein dudit gel.

Une étude de la rhéologie de différents mélanges aqueux gomme de caroube-acide polyacrylique réticulé a été réalisée. Cette étude montre l'évolution de l'indice de consisance et du seuil initial d'écoulement de ces mélanges, lorsque le taux de gomme de caroube augmente. On observe une augmentation significative de ces deux paramètres. Ceci se traduit par un comportement proche de celui du solide pour les gels à partir de 0,1 % en poids en gomme de caroube : ceux-ci sont plus rigides, plus cassants.

Le protocole et les résultats sont donnés ci-après : le comportement rhéologique des gels préparés comme à l'exemple 1 mais sans incorporation d'ingrédient actif ni d'additif, a été étudié en cisaillement continu à l'aide du système MS DIN 14 à 25 °C. On applique à l'échantillon un cycle de cisaillement pendant 2 minutes de 0 à 5 $s^{-1}$ et retour à 0. Les mesures sont effectuées plusieurs fois, pour deux passages successifs, et également complétées par une rampe de cisaillement de 5 à 500 $s^{-1}$. Les courbes d'écoulement obtenues sont exploitées avec le modèle d'HERSCHEL-BULKLEY, selon l'équation ci-après, afin de pouvoir être comparées entre elles :

$$\tau = \tau_0 + KD^n$$

où :
- K = indice de consistance ;
- n = indice d'écoulement ;
- $\tau_0$ = seuil initial ;

L'indice de consistance et le seuil initial sont les deux paramètres qui reflètent le caractère "solide" des mélanges étudiés.

TABLEAU I

| ETUDE DE RHEOLOGIE | | | |
|---|---|---|---|
| Acide polyacrylique réticulé (*) (% en poids) | Gomme de caroube (% en poids) | Indice de consistance K | Seuil initial 0 |
| 0,3 | - | 18,6 | 12,3 |
| 0,3 | 0,05 | 24,6 | 17,8 |
| 0,3 | 0,1 | 25 | 16,7 |
| 0,3 | 0,2 | 33,3 | 23 |
| 0,6 | - | 39,7 | 23,5 |
| 0,6 | 0,05 | 51,3 | 33,7 |
| 0,6 | 0,1 | 51 | 32,3 |
| 0,6 | 0,2 | 63,2 | 35,5 |
| Remarque : Les solutions de gomme de caroube à 0,05, 0,1 et 0,2 % en poids sont liquides. (A 1 % en poids, ce liquide est légèrement gélatineux). | | | |

(*) : Produit commercialisé sous la dénomination "CARBOPOL 940" par la Société "GOODRICH"

L'invention concerne donc des associations (polymère(s) carboxyvinylique(s)-galactomannane) permettant d'obtenir une augmentation de l'indice de consistance du gel de polymère d'au moins 10 % de sa valeur initiale.

On a également étudié le comportement de différents gels en présence de sels incompatibles avec les polymères à base de monomères carboxyvinyliques. Pour cela, à ces différents gels, préparés comme précédemment, on a ajouté du salicylate de triéthanolamine à 2,35 % en poids. Ce sel peut être utilisé comme bactériostatique ou comme solubilisant de certains principes actifs cosmétiques. Son incompatibilité avec les polymères de type précité entraîne une fluidification irréversible des gels, lesquels se troublent instantanément. Le tableau II ci-après présente les résultats des différents essais effectués :

TABLEAU II

| ETUDE DU COMPORTEMENT DES GELS EN PRESENCE DE SELS | | |
|---|---|---|
| Acide polyacrylique réticulé (*) (% en poids) | Gomme de caroube (% en poids) | Aspect du gel en présence du sel |
| 0,3 | - | Incompatibilité Produit trouble - sans viscosité |
| - | 0,013 | Aucune viscosité = Produit liquide Incompatibilité |
| 0,6 | - | Produit trouble - Fluide (Au bout de 2 mois, viscosité inchangée) Produit rigide et transparent |
| 0,6 | 0,013 | Viscosité : 12 poises (1,2 Pa.s) (Au bout de 2 mois, viscosité inchangée) Produit légèrement trouble |
| 0,9 | - | Tendance à l'écoulement -Viscosité : 22 poises (2,2 Pa.s) Gel rigide et cassant - |
| 0,9 | 0,013 | Viscosité : 29 poises (2,9 Pa.s) (Au bout de 2 mois, viscosité très augmentée) |

(*) : Produit commercialisé sous la dénomination de "CARBOPOL 940" par la société "GOODRICH"

On remarque qu'avec une quantité très faible de gomme de caroube (0,013 % en poids), les effets de cette incompatibilité sont supprimés, pour un taux moyen en polymère de 0,6 % en poids. On conclut à une meilleure résistance du polymère lorsqu'il est associé à la gomme de caroube.

La figure 1 du dessin annexé montre l'influence de l'addition de 0,15 % en poids de gomme de caroube à un gel à base d'acide polyacrylique réticulé (CARBOPOL 940), contenant 2,35 % en poids de salicylate de triéthanolamine. On a porté, en abscisse, le pourcentage en poids d'acide polyacrylique, et, en ordonnée, l'indice de consistance I selon Herschel-Bukley. Les courbes en trait discontinu et continu correspondent respectivement au gel sans addition et avec addition de gomme de caroube. La figure 2 montre la viscosité V (en poises ($= 10^{-1}$ Pa.s)) mesurée au viscosimètre Brookfield-Mobile 3, à 1 minute (zone hachurée) et à 10 minutes (zone non hachurée), en fonction du pourcentage en poids en gomme de caroube, d'une émulsion renfermant 2,35 % en poids de salicylate de triéthanolamine ainsi que 0,8 % d'acide polyacrylique réticulé (CARBOPOL 940).

Ces deux figures montrent donc que l'addition de gomme de caroube permet de compenser l'incompatibilité du sel employé avec l'acide polyacrylique réticulé, conduisant à une fluidification du gel à base dudit acide polacrylique. L'association de la gomme de caroube à celui-ci permet de renforcer fortement la viscosité apparente du gel.

Pour mieux faire comprendre l'objet de l'invention, on va en décrire maintenant, à titre d'exemples purement illustratifs et non limitatifs, plusieurs modes de mise en oeuvre.

EXEMPLE 1 : Gel amincissant

On réalise la formulation suivante :

| | |
|---|---|
| - Acide polyacrylique réticulé (PM 4 000 000), commercialisé sous la dénomination de "CARBOPOL 940" par la société "GOODRICH" | 0,6 g |
| - Gomme de caroube, commercialisée sous la dénomination de "GENU GUM RL 200" par la société "HERCULES" | 0,02 g |
| - Parahydroxybenzoate de méthyle | 0,02 g |
| - Extrait d'algue | 10 g |
| - Extrait de lierre | 10 g |
| - Imidazolidinyl urée, commercialisé sous la dénomination de "GERMALL 115" par la société "SUTTON LABS" | 0,2 g |
| - Triéthanolamine | 0,6 g |
| - Eau déminéralisée | qsp 100 g |

On fait gonfler dans 20 g d'eau, à chaud, l'acide polyacrylique réticulé, pour obtenir un liquide épais, auquel on ajoute le parahydroxybenzoate de méthyle, les extraits d'algue et de lierre et le conservateur, puis la triéthanolamine. On obtient un gel transparent.

Parallèlement, on fait gonfler, dans 10 g d'eau, à chaud, la poudre de gomme de caroube, pour obtenir un liquide blanchâtre.

On mélange le gel transparent et le liquide blanchâtre, on le dilue avec la quantité d'eau restante, et, au bout de quelques jours, on obtient un gel transparent et rigide.

EXEMPLE 2 : Gel contour des yeux

On réalise la formulation suivante :

| | |
|---|---|
| - Acide polyacrylique réticulé (PM 1 250 000), commercialisé sous la dénomination de "CARBOPOL 941" par la société "GOODRICH" | 0,7 g |
| - Gomme de caroube, commercialisée sous la dénomination de "GELLOID LB 100" par la société "MARINE COLLOIDS" | 0,015 g |
| - Caféine | 0,5 g |
| - Benzoate de sodium | 0,5 g |
| - Imidazolidinyl urée, commercialisé sous la dénomination de "GERMALL 115" par la société "SUTTON LABS" | 0,2 g |
| - Essence d'hamamelis | 0,015 g |
| - Triéthanolamine | 0,7 g |
| - Eau déminéralisée | qsp 100 g |

On procède comme à l'exemple 1, excepté que l'on incorpore la triéthanolamine après le mélange du gel transparent à base d'acide polyacrylique réticulé et du liquide blanchâtre constitué par la poudre de gomme de caroube gonflée dans l'eau à chaud.

On obtient également un gel transparent et rigide.

Les exemples 3 à 14 suivants concernent des compositions amincissantes comportant deux phases visqueuses (A) et (B), non-miscibles. La phase (A), dite interne, renferme le (ou les) principe(s) actif(s) et est dispersée, sous la forme d'inclusions, au sein de la phase (B), dite externe, laquelle est constituée par un gel conforme à la présente invention. La phase (A) doit avoir une viscosité qui est supérieure à 1 Pa.s (10 poises).

Pour fabriquer ces compositions, on a utilisé la machine telle que décrite dans le brevet français n°2 634 688, comprenant un plateau rotatif destiné à recevoir un pot pour contenir un gel et un produit d'inclusion, et des moyens pour injecter le produit d'inclusion dans le pot, avec possibilité d'un mouvement relatif entre le plateau et lesdits moyens pour injecter le produit suivant une direction sensiblement orthogonale au plateau. Initialement, les pots sont remplis de gel (phase (B)), de façon qu'aucune bulle d'air ne soit piégée dans la masse du gel ou à l'interface gel/paroi du pot.

EXEMPLE 3 : Produit amincissant de type gel à inclusions

On prépare une composition amincissante comportant, comme phase (A), une émulsion huile-dans-eau. Le rapport pondéral de la phase (A) à la phase (B) est de 10/90. Chacune des phases est formulée comme suit :

Phase interne (A) :

| | |
|---|---|
| - Cyclométhicone, commercialisé sous la dénomination de "VOLATIL SILICONE 7158" par la société "UNION CARBIDE" | 10 g |
| - Perhydrosqualène | 18 g |
| - Huile de vaseline | 5 g |
| - Lanoline liquide | 4 g |
| - Glycéryl stéarate + Polyéthylèneglycol (100 motifs) stéarate, commercialisé sous la dénomination "ARLACEL 165" par la société "ATLAS" | 6 g |
| - Produit commercialisé sous la dénomination "TWEEN 60" par la société "ICI" | 2 g |
| - Alcool cétylique | 1,2 g |
| - Acide stéarique | 2,5 g |
| - Triéthanolamine | 0,01 g |
| - Conservateur | 0,3 g |
| - Antioxydants | 0,3 g |
| - Eau déminéralisée | qsp 100 g |
| - Caféine | 1 g |
| - Extrait de Gingko biloba, commercialisé par la société "INVERNI" | 0,5 g |
| - Propylène glycol | 5 g |

On prépare une émulsion huile-dans-eau à partir de la formulation ci-dessus en procédant de la manière classique.

Phase (B)

| | |
|---|---|
| - Acide polyacrylique réticulé (PM 4 000 000), commercialisé sous la dénomination de "CARBOPOL 940" par la société "GOODRICH" | 0,3 g |
| - Gomme de caroube, commercialisée sous la dénomination de "GENU GUM RL 200-COPENHAGEN PECTIN" par la société "HERCULES" | 0,05 g |
| - Triéthanolamine | 0,3 g |
| - Eau déminéralisée stérile | qsp 100 g |
| - Conservateurs | 0,3 g |

On fait gonfler dans 15 g d'eau, à chaud, l'acide polyacrylique, puis on ajoute les conservateurs, puis la triéthanolamine dans le liquide épais obtenu, afin d'obtenir un gel transparent.

Parallèlement, on fait gonfler la poudre de gomme de caroube dans 10 g d'eau, à chaud, pour obtenir un liquide blanchâtre.

On mélange les deux compositions obtenues, on les dilue avec la quantité d'eau restante, et, au bout de quelques jours, on obtient un gel transparent et rigide.

On procède ensuite à l'injection, dans un pot de 150 ml à large ouverture, contenant la phase gel (B) ainsi obtenue, de la phase (A).

On obtient un aspect visuel attrayant. On laisse le pot reposer pendant 48 heures, pour permettre la stabilisation du gel transparent de phase (B).

Il n'est pas observé de déplacement de la phase (A) au sein de la phase (B), après un transport et lorsque le pot est soumis à des chocs légers.

EXEMPLE 4 : Produit amincissant de type gel à inclusions

On prépare une composition amincissante comportant, comme phase (A), une émulsion huile-dans-eau. La phase (B) est formulée comme à l'exemple 3. La phase (A) est formulée comme suit, le rapport pondéral de la phase (A) à la phase (B) étant de 15/85 :

Phase (A) :

| | |
|---|---|
| - Eau déminéralisée | qsp 100 g |
| - Propylène glycol | 2 g |
| - Polyéthylène glycol de masse moléculaire 400 | 3 g |
| - Extrait de Gingko biloba, commercialisé par la société "INVERNI" | 0,3 g |
| - Caféine | 3 g |
| - Benzoate de sodium | 3 g |
| - Conservateur | 0,3 g |
| - Parfum | 0,5 g |
| - Acide polyacrylique réticulé (PM 1 250 000), commercialisé sous la dénomination de "CARBOPOL 941" par la société "GOODRICH" | 0,2 g |
| - Myristate d'isopropyle | 1 g |
| - Alcool cétylique | 3 g |
| - Acide stéarique | 3 g |
| - Monostéarate de glycérol | 3 g |
| - Huile de germe de maïs | 2 g |

On réalise l'émulsion huile-dans-eau de la phase (A) de la façon classique. On prépare ensuite un pot de gel de phase (B) contenant des inclusions de phase (A), de la même façon qu'à l'exemple 3, et on effectue les mêmes constatations quant à la stabilité des inclusions de phase (A) au sein de la phase (B).

EXEMPLE 5 : Produit amincissant de type gel à inclusions

On prépare une composition amincissante comportant, comme phase (A), une émulsion eau-dans-huile. La phase (B) est identique à celle de l'exemple 3. Le rapport pondéral de la phase (A) à la phase (B) est de 1/99. La phase (A) est formulée comme suit :

Phase (A) :

| | |
|---|---|
| - Produit commercialisé sous la dénomination "PROTEGIN X" par la société "GOLDSCHMIDT" | 20 g |
| - Huile de vaseline | 10 g |
| - Composition aromatique | 1 g |
| - Huile de tournesol | 15 g |
| - Conservateur | 0,3 g |
| - Eau déminéralisée | qsp 100 g |
| - Glycérol Sulfate de magnésium | 5 g<br>0,5 g |
| - Extrait de lierre Saponnes totales, commercialisé par la société "INVERNI" | 0,5 g |
| - Produit commercialisé sous la dénomination "CETIOL HE" par la société "HENKEL" | 4 g |

On réalise l'émulsion eau-dans-huile de la phase (A) de la façon classique. On prépare ensuite un pot de gel de phase (B) contenant des inclusions de phase (A), de la même façon qu'à l'exemple 3, et on effectue les mêmes constatations quant à la stabilité des inclusions de phase (A) au sein de la phase (B).

EXEMPLE 6 : Produit amincissant de type gel à inclusions

On prépare une composition amincissante comportant, comme phase (A), une émulsion eau-dans-huile. La phase (B) est identique à celle de l'exemple 3. Le rapport pondéral de la phase (A) à la phase (B) est de 5/95. La phase (A) est formulée comme suit :

Phase (A) :

| | |
|---|---|
| - Produit commercialisé sous la dénomination "ABIL WE 09" par la société "GOLDSCHMIDT" | 5 g |
| - Myristate d'isopropyle | 5 g |
| - Cyclométhicone, commercialisé sous la dénomination "VOLATIF SILICONE 7188" par la société "UNION CARBIDE" | 8 g |
| Huile de vaseline | 5 g |
| - Silice colloïdale, commercialisé sous la dénomination "AEROSIL R 812" par la société "DEGUSSA AG" | 0,4 g |
| - Huile de purcellin, commercialisée par la société "DRAGOCOCO" | 14 g |
| - Eau déminéralisée | qsp 100 g |
| - Chlorure de sodium | 0,5 g |
| - Monométhyléther de diéthyléneglycol, commercialisé sous la dénomination "TRANSCUTOL" par la société "GATTEFOSSE" | 3 g |
| - Extrait de Gingko biloba, commercialisé par la société "INVERNI" | 0,5 g |
| - Caféine | 1 g |
| - Hydroxyde de sodium | 0,008 g |
| - Conservateur | 0,3 g |

On réalise l'émulsion eau-dans-huile de la phase (A) de la façon classique. On prépare ensuite un pot de gel de phase (B) contenant des inclusions de phase (A), de la même façon qu'à l'exemple 4, et on effectue les mêmes constatations quant à la stabilité des inclusions de phase (A) au sein de la phase (B).

EXEMPLE 7 : Produit amincissant de type gel à inclusions

On prépare une composition amincissante comportant, comme phase (A), un gel émulsionné huile-dans-eau. La phase (B) est identique à celle de l'exemple 3. Le rapport pondéral de la phase (A) à la phase (B) est de 10/90. La phase (A) est formulée comme suit :

Phase (A) :

| | |
|---|---|
| - Acide polyacrylique réticulé (PM 4 000 000), commercialisé sous la dénomination de "CARBOPOL 940" par la société "GOODRICH" | 0,6 g |
| - Cyclométhicone, commercialisé sous la dénomination "VOLATIL SILICONE 7158" par la société "UNION CARBIDE" | 3 g |
| - Huile de purcellin, commercialisée par la société "DRAGOCOCO" | 7 g |
| - Produit commercialisé sous la dénomination "TEFOSE 63" par la société "GATTEFOSSE" | 3 g |
| - Conservateur | 0,3 g |
| - Parfum | 0,4 g |
| - Alcool éthylique | 10 g |
| - Triéthanolamine | 0,2 g |
| - Eau déminéralisée | qsp 100 g |
| - Caféine | 1 g |
| - Produit commercialisé sou la dénomination "CETIOL HE" par la société "HENKEL" | 2 g |
| - Extrait standard de Gingko biloba, commercialisé par la société "INVERNI" | 0,8 g |

On réalise le gel émulsionné huile-dans-eau de la phase (A) de la façon classique. On prépare ensuite un pot de gel de phase (B) contenant des inclusions de phase (A), de la même façon qu'à l'exemple 4, et on effectue les mêmes constatations quant à la stabilité des inclusions de phase (A) au sein de la phase (B).

EXEMPLE 8 : Produit amincissant de type gel à inclusions

On prépare une composition amincissante comportant, comme phase (A), une dispersion aqueuse de vésicules à feuillets lipidiques non-ioniques encapsulant une phase aqueuse à teneur en agent actif. La phase (B) est la même que celle de l'exemple 3. Le rapport pondéral de la phase (A) à la phase (B) est de 25/75. La crème formant la phase (A) est préparée en deux étapes :

Première étape : Préparation d'une dispersion aqueuse de vésicules

On utilise une phase lipidique formulée comme suit :

| | |
|---|---|
| - Polyglycéryl-3 cétyl éther | 3,8 g |
| - $\beta$-sitostérol | 3,8 g |
| - Dicétyl-phosphate | 0,4 g |

On fond, en agitant doucement à une température de 95-100 °C, le mélange ayant la formulation ci-dessus, et, dans le mélange fondu, on introduit 16 g d'eau portés à 90 °C, sous agitation lente, après quoi on agite vivement dans une turbine munie de pales, jusqu'à obtention d'une masse gélifiée blanchâtre.

On ajoute alors 1,5 g de caféine et 16 g d'eau à 20 °C. On maintient la température et l'agitation pendant 60 min.

On affine le mélange.

A 40 °C, on introduit 10 g d'eau renfermant 0,3 g d'un conservateur, puis on affine à nouveau.

Deuxième étape : On réalise la formulation suivante :

| | |
|---|---|
| - Dispersion aqueuse de vésicules obtenues à la première étape | 50 g |
| - Benzoate de sodium | 1,5 g |
| - Monométhyléther de diéthylèneglycol, commercialisé sous la dénomination "TRANSCUTOL" par la société "GATTEFOSSE" | 3 g |
| - Escine acide, commercialisée par la société "INVERNI " | 0,5 g |
| - Hydroxyde de sodium | 0,007 g |
| - Extrait végétal hydrosoluble de lierre, commercialisé par la société "GATTEFOSSE" | 3 g |
| - Huile de tournesol | 35 g |
| - Parfum | 0,6 g |
| - Acide polyacrylique réticulé (PM 4 000 000), commercialisé sous la dénomination de "CARBOPOL 940" par la société "GOODRICH" (agent épaississant) | 0,02 g |
| - Triéthanolamine | 0,2 g |
| - Eau | qsp 100 g |

On introduit, dans la dispersion aqueuse de vésicules obtenue à la première étape, à 30 °C, l'huile de tournesol et du parfum, on agite en turbine et on affine.

On épaissit en dernier lieu le mélange en ajoutant un gel constitué par l'agent épaississant dissous dans l'eau.

On procède ensuite à l'injection de cette phase (A) dans la phase (B). On obtient un pot de produit amincissant à inclusions, sur lequel on fait les mêmes constatations que dans les exemples précédents, quant à la stabilité des inclusions.

EXEMPLES 9 à 14

On réalise la formulation suivante pour une phase (B) :

| | |
|---|---|
| - Acide polyacrylique réticulé (PM 1 250 000), commercialisé sous la dénomination de "CARBOPOL 941" par la société "GOODRICH" | 0,6 g |
| - Gomme de guar | 0,2 g |
| - Triéthanolamine | 0,6 g |
| - Eau déminéralisée stérile | qsp 100 g |
| - Conservateurs | 0,2 g |
| - Salicylate de triéthanolamine | 2,35 g |

On prépare un gel de phase (B) comme indiqué à l'exemple 3.

On procède comme aux exemples respectivement 3 à 8 en remplaçant la phase (B) de ces exemples par la phase (B) ci-dessus. On effectue les mêmes constatations quant à la rigidité du gel et au maintien des inclusions.

EXEMPLE 15

On a mesuré selon le modèle d'Herschel-Bulkley le seuil d'écoulement de gels préparés comme à l'exemple 1 mais, sans incorporation d'ingrédients actifs comme expliqué pour le tableau I, pour les compositions suivantes :

1 - un gel préparé avec 0,3 % en poids de polymère carboxyvinylique vendu sous la dénomination "CARBOPOL 940" par la société "GOODRICH"

2 - un gel préparé à partir de 0,3 % en poids de "CARBOPOL 940" et 0,1 % de gomme de caroube,

3 - un gel préparé à partir de 0,3 % en poids de "CARBOPOL 940" et 0,1 % de gomme de xanthane.

Les résultats sont donnés sur le graphique de la figure 3. On voit que l'addition de gomme de xanthane ne modifie pas le seuil d'écoulement du carbopol, il y a même au contraire fluidification, alors que l'addition de gomme de caroube modifie nettement ce seuil d'écoulement.

EXEMPLE 16

On a mesuré comme dans l'exemple 15, le seuil d'écoulement initial en pascals de compositions contenant des quantités croissantes de "CARBOPOL 940" et de gomme de caroube. Les résultats obtenus sont représentés sur la figure 4. On voit que l'addition de caroube permet d'augmenter le seuil d'écoulement de manière très nette.

EXEMPLE 17

On a également mesuré l'indice de consistance selon le modèle d'Herschel-Bulkley. Les résultats obtenus sont représentés sur la figure 5. On voit que l'indice de consistance d'un gel de caroube augmente significativement par addition d'une faible quantité de gomme de caroube.

**Revendications**

**1.** Composition cosmétique ou pharmaceutique, qui comprend au moins une phase gélifiée, rigide, donnant par cisaillement un produit doux et non collant, comportant, en tant qu'agent gélifiant, au moins un polymère carboxyvinylique, caractérisée par le fait qu'audit polymère carboxyvinylique est associé au moins un polygalactomannane.

**2.** Composition selon la revendication 1, caractérisée par le fait que le polygalactomannane est choisi dans le groupe formé par la gomme de caroube, la gomme de guar et la gomme de tara.

**3.** Composition selon l'une des revendications 1 et 2, caractérisée par le fait que le polymère carboxyvinylique est un acide polyacrylique réticulé, de masse moléculaire comprise entre 800 000 et 5 000 000.

**4.** Composition selon l'une des revendications 1 à 3, caractérisée par le fait que le(s) polymère(s) carboxyvinylique(s) est (ou sont) présent(s) à raison de 0,01 à 2 % en poids de la phase gélifiée qui le(s) contient.

**5.** Composition selon la revendication 4, caractérisée par le fait que le(s) polymère(s) carboxyvinylique(s) est (ou sont) présent(s) à raison de 0,1 à 1 % en poids de la phase gélifiée qui le(s) contient.

**6.** Composition selon l'une des revendications 1 à 5, caractérisée par le fait que le(s) polygalactomannane(s) est (ou sont) présent(s) à raison de 0,01 à 3 % en poids de la phase gélifiée qui le(s) contient, la quantité de polygalactomannane ajoutée étant telle que l'indice de consistance du polymère carboxyvinylique soit augmentée d'au moins 10 % par rapport à sa valeur obtenue avec le polymère carboxyvinylique seul.

**7.** Composition selon la revendication 6, caractérisée par le fait que le(s) polygalactomannane(s) est (ou sont) présent(s) à raison de 0,01 à 1.5 % en poids de la phase gélifiée qui le(s) contient.

**8.** Composition selon l'une des revendication 1 à 7, caractérisée par le fait qu'elle renferme des sels normalement incompatibles avec le(s) polymère(s) carboxyvinylique(s) choisi(s) dans le groupe des sels organiques formé par le salicylate de triéthanolamine, le benzoate de sodium et le salicylate de sodium.

**9.** Composition selon l'une des revendications 1 à 8, caractérisée par le fait qu'une phase gélifiée grâce au(x) polymère(s) carboxyvinylique(s) et au(x) polygalactomannane(s), contient au moins un ingrédient cosmétiquement ou pharmaceutiquement actif et/ou au moins un additif cosmétiquement ou pharmaceutiquement acceptable.

**10.** Composition selon l'une des revendications 1 à 9, consistant en au moins deux phases, caractérisée par le fait que l'une des phases est une phase continue dite "externe", gélifiée grâce au(x) polymère(s) carboxyvinylique(s), chaque autre phase étant une phase dite "interne", liquide ou visqueuse, se présentant sous la forme d'au moins une inclusion au sein de ladite phase externe.

**11.** Composition selon la revendication 10, caractérisée par le fait qu'une phase interne est constituée par une formulation aqueuse liquide, épaissie ou gélifiée, par une émulsion huile-dans-eau ou eau-dans-huile, par une dispersion aqueuse de vésicules constituées de feuillets lipidiques ioniques ou non-ioniques encapsulant une phase aqueuse, ou par des cristaux liquides.

**12.** Composition selon la revendication 11, caractérisée par le fait qu'une phase interne renferme au moins un ingrédient cosmétiquement ou pharmaceutiquement actif et/ou au moins un additif cosmétiquement ou pharmaceutiquement acceptable.

**13.** Composition selon l'une des revendications 10 à 12, caractérisée par le fait que la (ou les) phase(s) interne(s) représente(nt) de 0,1 à 25 % en poids de ladite composition.

**14.** Composition selon l'une des revendications 9 ou 12, caractérisée par le fait que le(s) ingrédient(s) actif-(s) est (sont) choisi(s) dans le groupe formé par les agents amincissants, les agents hydratants, les agents de fixation dans le domaine capillaire, les agents de protection solaire et les agents de soin de la peau et des cheveux.

**15.** Composition selon l'une des revendications 9 ou 12, caractérisée par le fait que le(s) additif(s) est(sont) choisi(s) dans le groupe formé par les agents émollients, les parfums, les agents modificateurs de pH, les agents conservateurs et les agents séquestrants.

**Claims**

**1.** Cosmetic or pharmaceutical composition which comprises at least one rigid gelled phase giving through shearing a mild and non-sticky product, containing, as gelling agent, at least one carboxyvinyl polymer, characterized in that at least one polygalactomannan is combined with the said carboxyvinyl polymer.

**2.** Composition according to Claim 1, characterized in that the polygalactomannan is chosen from the group consisting of carob gum, guar gum and tara gum.

**3.** Composition according to either of Claims 1 and 2, characterized in that the carboxyvinyl polymer is a cross-linked polyacrylic acid with a molecular mass of between 800,000 and 5,000,000.

**4.** Composition according to one of Claims 1 to 3, characterized in that the carboxyvinyl polymer(s) is (or are) present in an amount of 0.01 to 2% by weight of the gelled phase containing it (them).

**5.** Composition according to Claim 4, characterized in that the carboxyvinyl polymer(s) is (or are) present in an amount of 0.01 to 1% by weight of the gelled phase containing it (them).

**6.** Composition according to one of Claims 1 to 5, characterized in that the polygalactomannan(s) is (or are) present in an amount of 0.01 to 3% by weight of the gelled phase containing it (them), the quantity of polygalactomannan added being such that the consistency value of the carboxyvinyl polymer is increased by at least 10% relative to its value obtained with the carboxyvinyl polymer alone.

**7.** Composition according to Claim 6, characterized in that the polygalactomannan(s) is (or are) present in an amount of 0.01 to 1.5% by weight of the gelled phase containing it (them).

**8.** Composition according to one of Claims 1 to 7, characterized in that it contains salts normally incompatible with the carboxyvinyl polymer(s), chosen from the group of organic salts consisting of triethanolamine salicylate, sodium benzoate and sodium salicylate.

**9.** Composition according to one of Claims 1 to 8, characterized in that a phase gelled by means of the carboxyvinyl polymer(s) and the polygalactomannan(s) contains at least one cosmetically or pharmaceutically active ingredient and/or at least one cosmetically or pharmaceutically acceptable additive.

**10.** Composition according to one of Claims 1 to 9, consisting in at least two phases, characterized in that one of the phases is a continuous so-called "external" phase gelled by means of the carboxyvinyl

polymer(s), every other phase being a so-called liquid or viscous "internal" phase which is in the form of at least one inclusion inside the said external phase.

11. Composition according to Claim 10, characterized in that an internal phase consists of a thickened or gelled liquid aqueous formulation, an oil-in-water or water-in-oil emulsion, an aqueous dispersion of vesicles consisting of ionic or nonionic lipid layers encapsulating an aqueous phase, or liquid crystals.

12. Composition according to Claim 11, characterized in that an internal phase contains at least one cosmetically or pharmaceutically active ingredient and/or at least one cosmetically or pharmaceutically acceptable additive.

13. Composition according to one of Claims 10 to 12, characterized in that the internal phase(s) represents (represent) 0.1 to 25% by weight of the said composition.

14. Composition according to one of Claims 9 or 12, characterized in that the active ingredient(s) is (are) chosen from the group consisting of slimming agents, moisturizing agents, fixing agents in the hair domain, agents for protecting against sunlight and agents for care of the skin and the hair.

15. Composition according to one of Claims 9 or 12, characterized in that the additive(s) is (are) chosen from the group consisting of emollients, perfumes, pH modifiers, preservatives and sequestrants.

**Patentansprüche**

1. Kosmetische oder pharmazeutische Zusammensetzung, die wenigstens eine starre Gelphase umfaßt, welche bei Rühren ein weiches und nicht-klebriges Produkt ergibt und als gelbildendes Mittel wenigstens ein Carboxyvinyl-Polymer umfaßt, dadurch gekennzeichnet, daß mit dem Carboxyvinyl-Polymer wenigstens ein Polygalactomannan assoziiert ist.

2. Zusammensetzung nach Anspruch 1, dadurch gekennzeichnet, daß das Polygalactomannan ausgewählt ist unter Johannisbrotkernmehl, Guar- und Tara-Gummi.

3. Zusammensetzung nach einem der Ansprüche 1 und 2, dadurch gekennzeichnet, daß das Carboxyvinyl-Polymer eine vernetzte Polyacrylsäure mit einer Molekülmasse im Bereich von 800 000 bis 5 000 000 ist.

4. Zusammensetzung nach einem der Ansprüche 1 bis 3, dadurch gekennzeichnet, daß das (die) Carboxyvinylpolymer(e) in einem Anteil von 0,01 bis 2 %, bezogen auf das Gewicht der sie enthaltenden Gelphase, enthalten ist (sind).

5. Zusammensetzung nach Anspruch 4, dadurch gekennzeichnet, daß das (die) Carboxyvinylpolymer(e) in einem Anteil von 0,1 bis 1 %, bezogen auf das Gewicht der sie enthaltenden Gelphase, enthalten ist (sind).

6. Zusammensetzung nach einem der Ansprüche 1 bis 5, dadurch gekennzeichnet, daß das (die) Polygalactomannan(e) in einem Anteil von 0,01 bis 3 %, bezogen auf das Gewicht der sie enthaltenden Gelphase, enthalten ist (sind), wobei die zugesetzte Polygalactomannan-Menge so gewählt ist, daß der Viskositätsindex des Carboxyvinylpolymers um wenigstens 10 % des für reines Carboxyvinylpolymer erhaltenen Werts erhöht wird.

7. Zusammensetzung nach Anspruch 6, dadurch gekennzeichnet, daß das (die) Polygalactomannan(e) in einem Anteil von 0,01 bis 1,5 %, bezogen auf das Gewicht der sie enthaltenden Gelphase, enthalten ist (sind).

8. Zusammensetzung nach einem der Ansprüche 1 bis 7, dadurch gekennzeichnet, daß es Salze enthält, die normalerweise mit dem (den) gewählten Carboxyvinylpolymer(en) inkompatibel sind, ausgewählt unter den organischen Salzen Triethanolaminsalicylat, Natriumbenzoat und Natriumsalicylat.

9. Zusammensetzung nach einem der Ansprüche 1 bis 8, dadurch gekennzeichnet, daß eine aufgrund des (der) Carboxyvinylpolymers(e) und des (der) Polygalactomannan(e) gelierte Phase wenigstens einen kosmetisch oder pharmazeutisch aktiven Bestandteil und/oder wenigstens ein kosmetisch oder pharmazeutisch akzeptables Additiv enthält.

10. Zusammensetzung nach einem der Ansprüche 1 bis 9, bestehend aus wenigstens zwei Phasen, dadurch gekennzeichnet, daß eine dieser Phasen eine kontinuierliche, als "extern" bezeichnete Phase ist, die aufgrund des (der) Carboxyvinyl-Polymers(-Polymere) geliert ist, wobei jede andere Phase eine flüssige oder viskose "interne" Phase ist, die als wenigstens ein Einschluß im Inneren der externen Phase vorliegt.

11. Zusammensetzung nach Anspruch 10, dadurch gekennzeichnet, daß eine interne Phase gebildet wird von einer wäßrigen flüssigen, verdickten oder gelierten Formulierung, von einer Öl-in-Wasser- oder einer Wasser-in-Öl-Emulsion, von einer wäßrigen Dispersion von Vesikeln, bestehend aus ionischen oder nicht-ionischen Lipidschichten, die eine wäßrige Phase einschließen, oder von Flüssigkristallen.

12. Zusammensetzung nach Anspruch 11, dadurch gekennzeichnet, daß eine interne Phase wenigstens einen kosmetisch oder pharmazeutisch aktiven Bestandteil und/oder wenigstens ein kosmetisch oder pharmazeutisch akzeptables Additiv einschließt.

13. Zusammensetzung nach einem der Ansprüche 10 bis 12, dadurch gekennzeichnet, daß die interne(n) Phase(n) einem Anteil von 0,1 bis 25 %, bezogen auf das Gewicht der Zusammensetzung, entspricht (entsprechen).

14. Zusammensetzung nach einem der Ansprüche 9 oder 12, dadurch gekennzeichnet, daß der (die) aktive(n) Bestandteil(e) ausgewählt ist (sind) unter Verdünnungsmitteln, hydratisierenden Mitteln, fixierenden Mitteln für Haar, Sonnenschutzmitteln und Pflegemitteln für Haut und Haar.

15. Zusammensetzung nach einem der Ansprüche 9 oder 12, dadurch gekennzeichnet, daß das (die) Additiv(e) ausgewählt ist (sind) unter weichmachenden Mitteln, Parfüms, den pH-Wert modifizierenden Mitteln, Konservierungsmitteln und Sequestriermitteln.

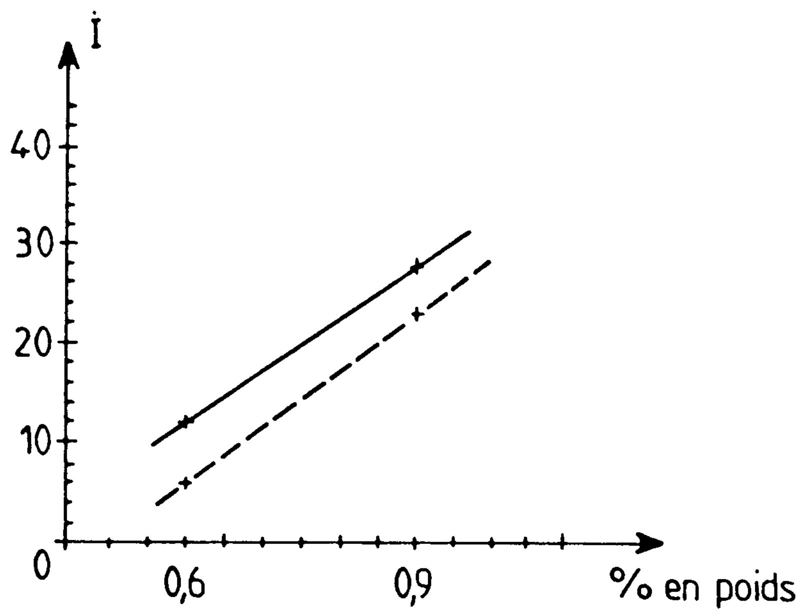


FIG. 1

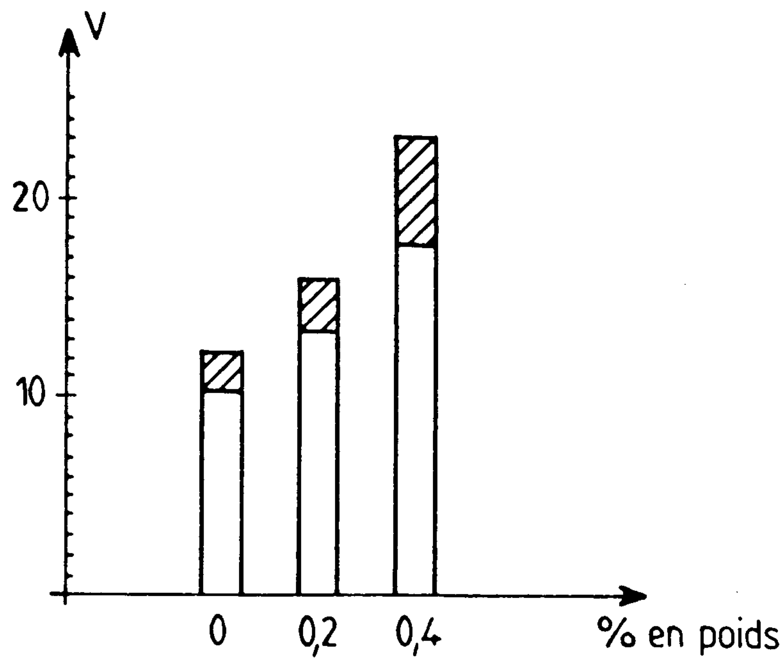


FIG. 2

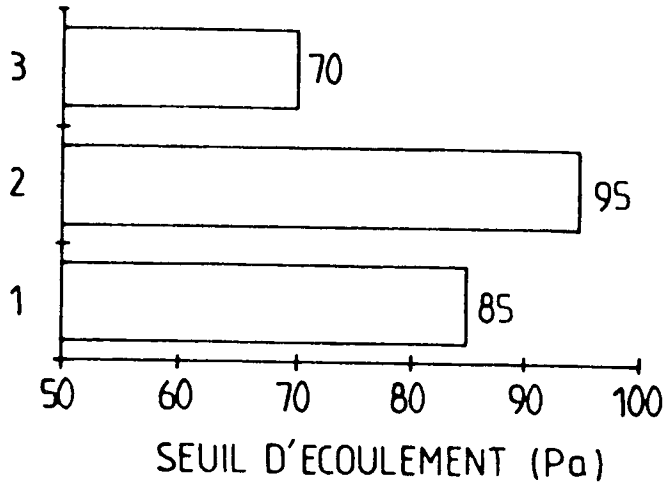


FIG. 3

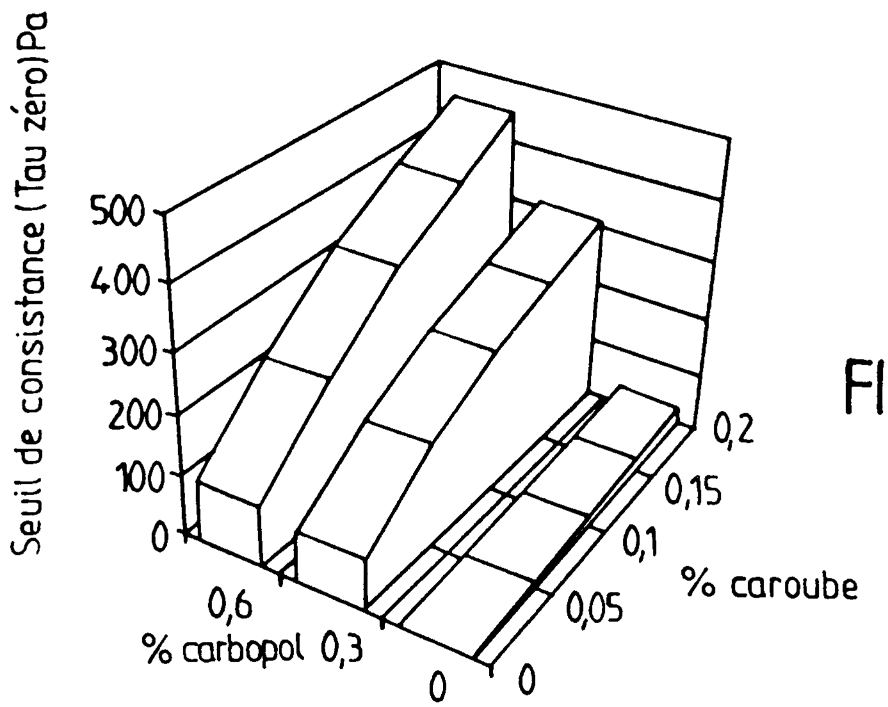


FIG. 4

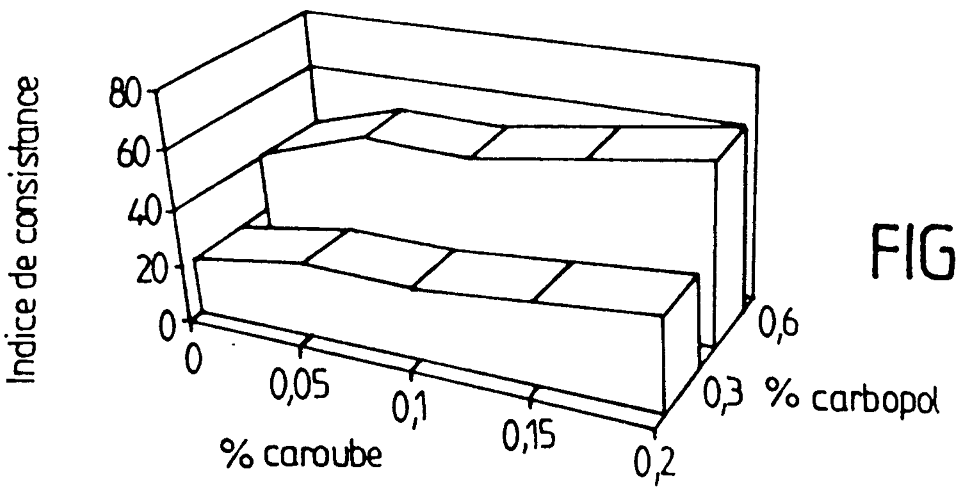


FIG. 5